(19) Europäisches Patentamt
European Patent Office
Office européen des brevets

(11) Veröffentlichungsnummer: **0 293 563 A1**

# (12) EUROPÄISCHE PATENTANMELDUNG

(21) Anmeldenummer: **88103748.5**

(22) Anmeldetag: **09.03.88**

(51) Int. Cl.4: **A61K 35/78 , //(A61K35/78, 31:60),(A61K35/78,31:195), (A61K35/78,31:19),(A61K35/78, 31:42),(A61K35/78,31:40), (A61K35/78,31:505), (A61K35/78,31:10),(A61K35/78, 31:38),A61K35:78,A61K31:415, A61K35:78,A61K31:53**

(30) Priorität: **09.03.87 DE 3707532**

(43) Veröffentlichungstag der Anmeldung: **07.12.88 Patentblatt 88/49**

(84) Benannte Vertragsstaaten: **AT BE CH DE ES FR GB GR IT LI LU NL SE**

(71) Anmelder: **OXO-Chemie GmbH**
**Maassstrasse 24**
**D-6900 Heidelberg(DE)**

(72) Erfinder: **Bauer, Johann Dr. med.**
**Volkartstr. 64**
**DE-8000 Muenchen 19(DE)**

(74) Vertreter: **Spott, Gottfried, Dr. et al**
**Patentanwälte Spott und Puschmann**
**Sendlinger-Tor-Platz 11**
**D-8000 München 2(DE)**

## (54) Kombinationspräparat, insbesondere gegen entzündliche Prozesse.

(57) Beschrieben wird ein Kombinationspräparat, insbesondere gegen entzündliche Prozesse, welches eine Kombination von Ginkgo biloba oder mindestens einem Gingkolid und mindestens einem Entzündungshemmer enthält. Dieses Präparat eignet sich vor allem zur Erstbehandlung von Verbrennungen unter Einschluß von Sonnenbrand, Verbrühungen und Strahlenschäden sowie von Kombinationstraumata, Erfrierungen, Schockzuständen, Sepsis, Pankreatitis, Tourniquet Syndrom und Palacosreaktion.

EP 0 293 563 A1

## Kombinationspräparat, insbesondere gegen entzündliche Prozesse

Die Erfindung betrifft ein Kombinationspräparat, insbesondere für die Behandlung von entzündlichen Prozessen.

Bei der Behandlung von entzündlichen Prozessen, wie sie insbesondere als Folgeerscheinungen von Verbrennungen auftreten und mit Störungen der Blutbestandteile, insbesondere der Thrombozyten, einhergehen, wurde bereits vorgeschlagen (EP-A-0 169 466), eine Kombination von Xanthinderivaten mit Acetylsalicylsäurederivaten einzusetzen. Diese Kombination zeigte sich, insbesondere wenn sie in zeitlicher Abfolge gegeben wurde, als wirksam gegen Störungen des Gleichgewichts der Blutbestandteile, wie sie beispielsweise bei Verbrennungen auftreten.

Diese bekannte Kombination ist jedoch in Fällen, bei denen eine Acetylsalicylsäure-Unverträglichkeit oder auch eine Unverträglichkeit der Xanthinderivate auftritt, nicht oder schlecht einsetzbar.

Es ist daher Aufgabe der Erfindung, ein Präparat zu schaffen, das die Entzündungskaskade beziehungsweise die biochemischen entzündlichen Reaktionen, wie sie beispielsweise bei einer akuten Verbrennung entstehen, hemmt und auch in Fällen eingesetzt werden kann, in denen eine Unverträglichkeit der aus der EP-A-0 169 466 bekannten Kombinationspräparate auftritt, bei dem zudem eine Beachtung irgendwelcher zeitlicher Abfolge unnötig ist. Es dient auch der perioperativen Entzündungsprophylaxe und damit als perioperative Infektionsprophylaxe. Es läßt Schmerzmittel und Antibiotika einsparen, die als perioperative Prophylaxe oder bei eintretendem Infekt gegeben werden. Auch als Mittel gegen den Sonnenbrand ist es parenteral, oral oder topisch einsetzbar.

Die Aufgabe wird erfindungsgemäß gelöst durch ein Kombinationspräparat, insbesondere gegen entzündliche Prozesse, das gekennzeichnet ist durch eine Kombination von Extrakt Ginkgo biloba oder mindestens einem Ginkgolid und mindestens einem Entzündungshemmer.

Dabei wird unter Ginkgolid hier der wirksame Bestandteil des Extrakts Ginkgo biloba, der die Thrombozytenaggregation beeinflußt, verstanden.

Das Kombinationspräparat kann bei einer besonders bevorzugten Ausführungsform der Erfindung eine oder mehrere Substanzen enthalten, die aus der Gruppe der Salicylsäure und ihrer Derivate, wie DL-Lysin-mono-(acetylsalicylat) und deren fluorsubstituierten Derivaten, beispielsweise Diflunisal, ausgewählt sind.

Es kann aber auch mindestens eine Substanz aus der Gruppe der Fenac-Wirkstoffe, wie Diclofenac, Biphenylessigsäure, Fenbufen, als NSAID einzeln oder in Kombination mit einem anderen NSAID eingesetzt werden, wie mindestens eine Substanz aus der Profen-Gruppe, wie Benoxaprofen, Carprofen, Fenoprofen, Flurbiprofen, Ibuprofen, Indoprofen, Ketoprofen, Naproxen, Purprofen oder Proquazon. Es eignen sich auch Indenessigsäurederivate, insbesondere zur "trans"-Indometacinanordnung osostere Systeme, wie Slindac oder Sulindacsulfid (prodrug Sulindac).

Das erfindungsgemäße Kombinationspräparat kann auch Dimethylsulfoxid oder andere Sulfoxide und/oder auch Substanzen, die ausgewählt sind aus der Gruppe der Pyrrol-, Thiophen- und Pyrazolonessigsäuren, wie Tolmetin, Zomepirac, Tiaprofensäure oder Lozanolac, oder/und auch entzündungshemmende heterocyclische Enole, die ausgewählt sind aus der Gruppe der Pyrazolidinderivate,. wie Oxyphenbutazon, Azapropazon, Perclusone, Tonorac, Suxibuon oder Bumadizon, oder Derivate von 1,2-Benzothiazin-3-carbonsäureamid, wie Piroxicam, enthalten.

Das erfindungsgemäße Kombinationspräparat liegt zur schnellen Verabreichung bevorzugt in einer Injektionslösung vor, die gegebenenfalls ein übliches Trägermaterial enthält. Für spezielle Lagerbedingungen (Erste-Hilfe-Kästen oder dergleichen) kann es jedoch auch in Tablettenform bei geeigneten Wirkstoffkombinationen gegeben werden.

Insbesondere eignet sich das erfindungsgemäße Kombinationspräparat zur Verwendung bei der Erstbehandlung von Verbrennungen für medizinische und tiermedizinische Zwecke und bei allen Schäden, die ohne Rücksicht auf das verursachende Läsionsprinzip die Entzündungskaskade in Gang setzen.

Das Kombinationspräparat kann auch ein Xanthinderivat der allgemeinen Formel

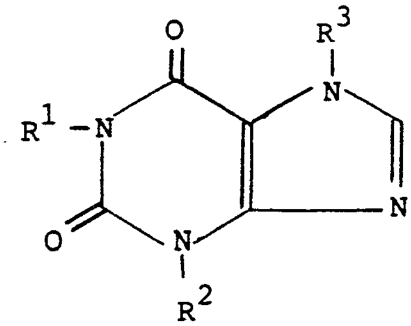

in der einer der Reste $R^1$ und $R^3$ eine geradkettige Alkyl-, (Omega-1)-Oxoalkyl- oder (Omega-1)-Hydroxyalkylgruppe mit 3 bis 8 Kohlenstoffatomen bedeutet und die beiden anderen Reste $R^2$ und $R^3$ oder $R^1$ und $R^2$ geradkettige oder verzweigte Alkylgruppen mit 1 bis 8 Kohlenstoffatomen in der Position von $R^1$ und $R^3$ und mit 1 bis 4 Kohlenstoffatomen in der Position von $R^2$ darstellen, wobei die Summe der Kohlenstoffatome dieser beiden Alkylsubstituenten höchstens 10 beträgt, oder auch ein Xanthinderivat der allgemeinen Formel

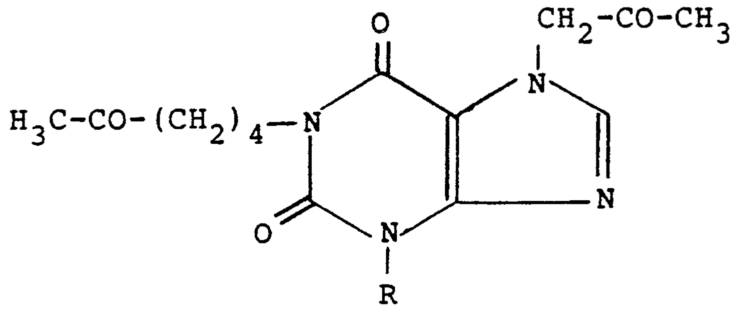

in der R für einen Alkylrest mit 1 bis 4 Kohlenstoffatomen steht, oder auch Prodrug-Formen der oben genannten Oxoalkylxanthine oder deren Metaboliten enthalten.

Ferner kann dieses Präparat mindestens einen natürlichen, organisch vorkommenden Sauerstoffradikalfänger, wie Gallussäure, enthalten.

Es kann auch bevorzugt sein, daß dieses Kombinationspräparat weiter ein gerinnungshemmendes Mittel, wie Heparin, aufweist.

Vorteilhafterweise kann das Kombinationspräparat auch noch ein durchblutungsförderndes Mittel, wie Nicergolin, aufweisen, das ebenfalls eine Wirkung auf die Blutplättchen ausübt.

Es hat sich überraschenderweise gezeigt, daß auch die Kombination von Hemmstoffen für verschiedene Teilbereiche der bei entzündlichen Prozessen ablaufenden Entzündungskette mit einem die Thrombozytenaggregation (platelet activating factor wird gehemmt) hemmenden Wirkstoff, wie einen Gingkoextrakt (beispielsweise den unter den Warenzeichen RÖKAN von der Firma Intersan, Ettlingen, oder TEBONIN von der Firma W. Schwabe, Karlsruhe, als durchblutungsfördernde Mittel vertriebenen Präparaten) bei Untersuchungen an Schweinen mit Verbrennungen und auch bei menschlichen Opfern von Verbrennungen und/oder Schockzuständen bei akuter Gabe dieser Wirkstoffe, bevorzugt in Form einer "one-shot Injektion", das Auftreten von teilweise letalen Spätfolgen überschiessender biochemischer entzündungsartiger Reaktionen, wie ARDS (Lungenschäden), Verbrennungskrankheit und Sepsis, zu verhindern vermögen und auch bei Pankreatitis, akutem Herzinfarkt und allen anderen akuten Ereignissen, die auf einem völligen Ungleichgewicht des Midiatorenspiegels beruhen, abzufangen und therapeutisch zu steuern vermögen.

Es ist nun überraschenderweise gefunden worden, daß auch die Kombination von Ginkgo biloba Extrakten und/oder Pentoxifillin mit einem NSAID dazu geeignet ist, ein Überschießen der Entzündungsprozesse zu stoppen und in vielen Fällen lebensrettend zu wirken, falls das Präparat in einem hinreichend kurzen Zeitraum nach einem Ereignis, wie einer Verbrennung, als erste Hilfe gegeben werden kann.

Es wird angenommen, daß es sich hier um ein frühes Eingreifen in die Entzündungskette unter anderem durch die Hemmung von PAF (platelet activating factor) durch den Gingko biloba Extrakt beziehungsweise durch dessen wirksame Bestandteile und um eine Cyclooxygenasehemmung oder eine Lipoxygenasehemmung (Inhibierung eines Teils der Arachidonsäurekaskade), je nach eingesetztem Entzündungshemmer, handelt.

Nachfolgend soll die Erfindung anhand von Beispielen näher erläutert werden, die die überraschende Wirkung des erfindungsgemäßen Kombinationspräparats zeigen.

Verbrühungsversuch:

Deutsche Hausschweine mit einem Körpergewicht von 17 bis 20 kg beiderlei Geschlechts, die mit standardisiertem Schweinefutter und Wasser ad libitum unter klimatisierten Bedingungen (Raumtemperatur 20°C + 2°C, Luftfeuchtigkeit 55 % + 5 %, Lichtwechselfrequenz 12 Stunden) gehalten wurden, wurden als Versuchstiere für einen Verbrühungsversuch eingesetzt. Aus chronobiologischen Gründen erfolgte das Setzen der Läsion jeweils vormittags gegen 9.00 Uhr.

Die Tiere erhielten zur Narkoseeinleitung 5 ml Vetranquil intramuskulär, worauf eine intravenöse Nadel in die Ohrvene eingestochen wurde. Die Tiere erhielten dann intravenös 1,5 ml mit physiologischer Kochsalzlösung verdünntes Narkoren. Erforderlichenfalls wurde in zeitlichen Abständen 0,5 ml verdünntes Narkoren bis zu einer Gesamtdosis von umgerechnet 5 mg Narkoren nachgespritzt. Die Tiere wurden an Hals, Rücken und Flanke rasiert und am Hals und Nacken operiert. In die Jugularis interna wurde ein Katheter für die Blutentnahme für die notwendigen biochemischen Untersuchungen eingelegt. Der Katheter wurde subcutan am Nacken ausgeleitet und festgenäht. Zum Abgrenzen der Läsion wurde das zu verbrühende Areal an den Rändern mit Klebestreifen abgedeckt. Das narkotisierte Tier wurde auf die Seite gelegt und mit der Flanke derart in einen Verbrühungsbehälter mit einer 15 x 45 cm großen Öffnung gelegt, daß etwa 20 % der Hautoberfläche verbrüht wurden. Die Eintauchzeit in das konstant bei 75°C gehaltene Bad betrug jeweils 10 Sekunden. Es wurde eine Verbrühung des Grades II halten. In drei Fällen wurden auch Verbrühungen des Grades III durch Erhöhen der Temperatur auf 90°C und Verlängern der Eintauchzeit auf 15 Sekunden durchgeführt.

Drei Tiere einer Kontrollgruppe wurden verbrüht und erhielten 5 Minuten nach Läsion nur 2 x 50 ml physiologische Kochsalzlösung über den Katheter langsam (30 Min.) einperfundiert. Drei Tiere der ersten Therapiegruppe erhielten 5 Minuten nach der Verbrühung II b eine 25 ml Ampulle handelsüblichen Tebonins (eine Infusionsampulle (25 ml) enthält Extrakt Fol. Ginkgo biloba sicc. puriss. pro Injektion 87,5 mg, standardisiert auf Ginkgoflavonglykoside 21 mg, Sorbit 1 g) in 50 ml NaCl über den Katheter perfundiert und gleichzeitig eine Ampulle Aspisol (eine Injektionsflasche enthält 0,9 g DL-Lysinmonoacetylsalicylat, entsprechend 0,5 g Acetylsalicylsäure,und 0,1 g Aminoessigsäure) in 50 ml physiologischem Natriumchlorid über den gleichen Katheter. Das gleiche erhielten drei Tiere der zweiten Therapiegruppe 5 Minuten nach Verbrühung III.

Im Gegensatz zur Kontrollgruppe zeigten die drei Tiere der ersten Therapiegruppe eine lebhafte, livide Verfärbung der verbrühten Areale an den Stellen tiefdermaler Läsionen (II b/a). An den drittgradig verbrühten Arealen entwickelten die Tiere der zweiten Therapiegruppe einen glänzenden trockenen und nicht wie bei den Tieren der Kontrollgruppe nässenden Belag, der durchsichtig glasurartig war und die in der Subcutis gelegenen Gefäße erkennen ließ. Diese Gefäße waren durchblutet, wobei durch Fingerdruck die Durchblutung reversibel weggedrückt werden konnte. Die am 7. Tag nach der Verbrühung entnommenen histologischen Präparate aus den Verbrühungsarealen zeigten eine bessere Wundheilung als bei den Tieren der Kontrollgruppe. Die Therapiegruppen zeigten auch eine Aufhebung der Peaks bei den Verläufen der Prostanoidspiegel im Indomethacinplasma.

Im Gegensatz dazu zeigten die unbehandelten Tiere der Kontrollgruppe nässende, schlechter heilende Wunden und eitrigen Schorf. Die Peaks bei den Verläufen der Prostanoidspiegel im Indomethacinplasma waren voll ausgebildet.

Aus den morphologischen und biochemischen Befunden läßt sich ableiten, daß die Gabe des erfindungsgemäßen Kombinationspräparates deutlich positive Ergebnisse in bezug auf die Wundheilung und die biochemischen Mechanismen hervorruft.

Aus diesen Gründen wurde bei mehreren Patienten eine "one-shot"-Therapie durchgeführt. Insgesamt 24 Patienten mit Verbrennungen erhielten eine Ginkgo-Acetylsalicylsäure-Infustion und diese Medikation wurde oral für eine Woche fortgeführt.

Jeweils 12 Patienten mit Verbrennungen erhielten eine "one-shot"-Infusion, bestehend aus 10 mg Ginkgoflavonglykoside und 500 mg Sorbit, 0,450 g DL-Lysinmonoacetylsalicylat und 0,05 g Aminoessigsäure sowie 150 mg Pentoxifyllin in 500 ml isotoner-isoioner Vollelektrolytlösung zur intravenösen Infusion - aber ohne Weiterführung der Therapie.

In beiden Patientengruppen gaben die Patienten, die bei Bewußtsein waren, bereits 10 Minuten nach Infusionsbeginn an, daß die Schmerzen nachließen. Es konnte auf Schmerzmittel aus der Btm-Liste, die bei

Verbrennungen sonst üblich sind, verzichtet werden. Gerötete Hautareale blaßten ab, schwerer geschädigte Areale mit Blasenbildung färbten sich livide, drittgradig verbrannte Areale bildeten nach 60 Minuten den (aus den Tierversuchen bekannten) glasurartigen Schorf. Dieser Schorf blieb trocken und infektfrei und bedurfte keiner besonderen Verbände. Er entsprach einer Gerbung. Hierbei handelt es sich um einen durch Medikation induzierten Vorgang, so daß durchaus von einer inneren Gerbung gesprochen werden kann.

Der glasurartige Schorf entsprach exakt den mittels Sonographie ermittelten Arealen drittgradiger Läsion. Diese wurden am dritten Tag exzidiert und mit mesh-graft gedeckt. Hervorzuheben ist das problemlose Einheilen der mesh-grafts, insbesondere auch das Fehlen von Infektionszeichen und Atmungsproblemen bei den Patienten.

Extremes Beispiel war eine 88-jährige Patientin, die mit Inhalationstrauma und 40 % Verbrennung eingeliefert wurde. Davon waren 20 % drittgradig verbrannt und exzisionswürdig. Die Patientin wurde, wie beschrieben, behandelt. Die mesh-grafts heilten primär, die Patientin hatte nie Atmungsprobleme und überlebte in gutem Allgemeinzustand.

Aufgrund dieser erstaunlichen Resultate wurden zwei Patienten mit beginnendem septischen Schock mit der Ginkgo-Acetylsalicylsäure/Pentoxifyllin-Kombination als "one-shot"-Therapie behandelt. Der septische Schock konnte abgefangen werden.

Ein Patient, der mit Blutdruckwerten systolisch unter 60 mm/Hg übernommen wurde, zeigte als Krankheitsursache einen akuten Schub einer Pankreatitis mit Arrosion der Milzarterie und Einblutung in eine Pankreaspseudocyste. Auch er erhielt das Kombinationspräparat, erholte sich innerhalb einer Stunde und konnte am dritten Tag erfolgreich operiert und saniert werden.

Auch ein Kleinkind wurde perioperativ mit dem Kombinationspräparat behandelt (die Dosierung wurde auf das Körpergewicht umgerechnet), als es wegen einer Förderbandverletzung beider Hände (Verbrennung durch die Reibungshitze, Quetschungen und Amputationen) versorgt werden mußte. Die operativen, rekonstruktiven Maßnahmen heilten ohne Infektion primär und ästhetisch zufriedenstellend.

Durch das neue Kombinationspräparat wird die Startstrecke jeder Entzündungsreaktionen - unabhängig vom Auslösemechanismus - so gestört, daß die kritische Masse an Mediatoren nicht erreicht wird und die letale unkontrollierbare Kettenreaktion zur gesteuerten behebbaren Störung des Mediatorenspiels gezügelt wird, mit welcher ein Organismus fertig werden kann.

**Ansprüche**

1. Kombinationspräparat, insbesondere gegen entzündliche Prozesse, gekennzeichnet durch eine Kombination von Extrakt Ginkgo biloba oder mindestens einem Gingkolid und mindestens einem Entzündungshemmer.

2. Kombinationspräparat nach Anspruch 1, dadurch gekennzeichnet, daß der Entzündungshemmer ein NSAID (non steroidal antiinflammatory drug) ist.

3. Kombinationspräparat nach Anspruch 2, dadurch gekennzeichnet, daß das NSAID Acetylsalicylsäure oder eines ihrer Derivate, wie DL-Lysin-mono-(acetylsalicylat) und fluorsubstituierte Derivate hiervon, beispielsweise Diflunisal, ist.

4. Kombinationspräparat nach einem der vorhergehenden Ansprüche, dadurch gekennzeichnet, daß es mindestens eine Substanz aus der Gruppe der Fenac-Wirkstoffe, wie Diclofenac, Biphenylessigsäure oder Fenbufen, enthält.

5. Kombinationspräparat nach einem der vorhergehenden Ansprüche, dadurch gekennzeichnet, daß es mindestens eine Substanz aus der Profen-Gruppe, wie Benoxaprofen, Carprofen, Fenoprofen, Flurbiprofen, Ibuprofen, Indoprofen, Ketoprofen, Naproxen, Purprofen oder Proquazon, enthält.

6. Kombinationspräparat nach einem der vorhergehenden Ansprüche, dadurch gekennzeichnet, daß es mindestens ein Indenessigsäurederivat, insbesondere ein zur "trans"-Indometacinanordnung isosteres System, beispielsweise Sulindac oder Sulindacsulfid (prodrug Sulindac), enthält.

7. Kombinationspräparat nach einem der vorhergehenden Ansprüche, dadurch gekennzeichnet, daß es Dimethylsulfoxid oder andere Sulfoxide enthält.

8. Kombinationspräparat nach einem der vorhergehenden Ansprüche, dadurch gekennzeichnet, daß es eine Substanz, ausgewählt aus der Gruppe der Pyrrol-, Thiophen- und Pyrazolonessigsäuren, wie Tolmetin, Zomepirac, Tiaprofensäure oder Lozanolac, enthält.

9. Kombinationspräparat, dadurch gekennzeichnet, daß es ein entzündungshemmendes heterocyclisches Enol aus der Gruppe der Pyrazolidinderivate, wie Oxyphenbutazon, Azapropazon, Perclusone, Ranorac, Suxibuzon oder Bumadizon, oder ein Derivat von 1,2-Benzothiazin-3-carbonsäureamid, wie Piroxicam, enthält.

10. Kombinationspräparat, dadurch gekennzeichnet, daß es in einer Injektionslösung, gegebenenfalls zusammen mit einem Trägermaterial, vorliegt.

11. Kombinationspräparat nach einem der vorhergehenden Ansprüche zur Verwendung bei der Erstbehandlung von Verbrennungen (eingeschlossen Sonnenbrand), Verbrühungen und Strahlenschäden sowie bei Kombinationstraumata, Erfrierungen, Schockzuständen, Sepsis, Pankreatitis, Tourniquet Syndrom und Palacosreaktion.

12. Kombinationspräparat nach einem der vorhergehenden Ansprüche, dadurch gekennzeichnet, daß es ferner ein Xanthinderivat der allgemeinen Formel

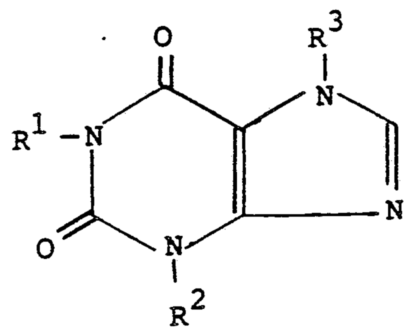

in der einer der Reste $R^1$ und $R^3$ eine geradkettige Alkyl-(Omega-1)-Oxoalkyl- oder (Omega-1)-Hydroxyalkylgruppe mit 3 bis 8 Kohlenstoffatomen bedeutet und die beiden anderen Reste $R^2$ und $R^3$ oder $R^1$ und $R^2$ geradkettige oder verzweigte Alkylgruppen mit 1 bis 8 Kohlenstoffatomen in der Position von $R^1$ und $R^3$ und mit 1 bis 4 Kohlenstoffatomen in der Position von $R^2$ darstellen, wobei die Summe der Kohlenstoffatome dieser beiden Alkylsubstituenten höchstens 10 beträgt, oder ein Xanthinderivat der allgemeinen Formel

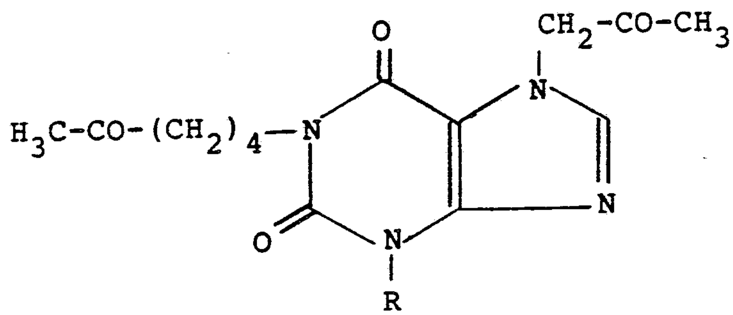

in der R für einen Alkylrest mit 1 bis 4 Kohlenstoffatomen steht, oder auch Prodrug-Formen der oben genannten Oxoalkylxanthine oder deren Metaboliten enthält.

13. Kombinationspräparat nach einem der vorhergehenden Ansprüche, dadurch gekennzeichnet, daß es ferner mindestens einen natürlichen, organisch vorkommenden Sauerstoffradikalfänger, wie Gallussäure, enthält.

14. Kombinationspräparat nach einem der vorhergehenden Ansprüche, dadurch gekennzeichnet, daß es ferner ein gerinnungshemmendes Mittel, wie Heparin, enthält.

15. Kombinationspräparat nach einem der vorhergehenden Ansprüche, dadurch gekennzeichnet, daß es ein durchblutungsförderndes Mittel, wie Nicergolin, enthält.

Europäisches Patentamt

# EUROPÄISCHER RECHERCHENBERICHT

Nummer der Anmeldung

EP 88 10 3748

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | PROSTAGLANDINS, Band 32, Nr. 5, November 1986, Seiten 719-727, Los Altos, California, US; R.S.B. CORDEIRO et al.: "Salicylates inhibit PAF-acether-induced rat paw oedema when cyclooxygenase inhibitors are ineffective" * Seiten 719,720,725 * | 1-3 | A 61 K 35/78 // (A 61 K 35/78 A 61 K 31:60 ) (A 61 K 35/78 A 61 K 31:195) (A 61 K 35/78 A 61 K 31:19 ) (A 61 K 35/78 A 61 K 31:42 ) (A 61 K 35/78 A 61 K 31:40 ) (A 61 K 35/78 A 61 K 31:505) (A 61 K 35/78 A 61 K 31:10 ) (A 61 K 35/78 A 61 K 31:38 ) -/- |
| A | FR-A-2 583 640 (P. BERDAL) * Ansprüche 1-3 * | 1,12,14 | |
| A | EP-A-0 174 006 (RICHARDSON-VICKS) * Ansprüche 1,8-11 * | 1-9 | |
| | | | RECHERCHIERTE SACHGEBIETE (Int. Cl.4) |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 27-09-1988 | PEETERS J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 88 10 3748

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | | | A 61 K 35:78<br>A 61 K 31:415<br>A 61 K 35:78<br>A 61 K 31:53<br>A 61 K 35:78<br>A 61 K 31:54 |
| | | | RECHERCHIERTE SACHGEBIETE (Int. Cl.4) |
| | | | |

Der vorliegende Recherchenbericht wurde für alle Patentansprüche erstellt

| Recherchenort | Abschlußdatum der Recherche | Prüfer |
|---|---|---|
| DEN HAAG | 27-09-1988 | PEETERS J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)